# EUROPEAN PATENT APPLICATION

(11) **EP 0 931 530 A1**
(43) Date of publication of application: **28.07.1999**
(21) Application number: 98101175.2
(22) Date of filing: 23.01.1998
(51) Int. Cl.: A61F 13/15, A61F 13/56

(54) **Incontinence pad having improved securement system**

(71) Applicant: THE PROCTER & GAMBLE COMPANY, Cincinnati, Ohio 45202 (US)
(72) Inventor: Heinermann, Gregor Heribert, 65239 Hochheim - Massenheim (DE)
(74) Representative: Canonici, Jean-Jacques

(57) **Abstract**

An incontinence pad which is to be secured to a pant. The incontinence pad has a pair of end edges and a pair of side edges, a liquid pervious topsheet, a liquid impervious backsheet secured to the topsheet, and an absorbent core intermediate the topsheet and the backsheet. A pair of spaced apart securement patches are positioned adjacent each end edge of the incontinence pad. A unitary release strip bridges the securement patches.

## Description

### FIELD OF THE INVENTION

The present invention relates to articles which absorb and/or contain bodily exudates. More particularly, the invention relates to disposable incontinence pads having an improved securement system.

### BACKGROUND OF THE INVENTION

Disposable incontinence pads used in combination with reusable pants are well known in the art. Also, the securement of such incontinence pads to the pants is also known in the art.

In particular, incontinence pads having a pair of adhesive patches positioned adjacent each end edge of the pad have been found to be effective in securing the incontinence pad to the pant. Typically, each adhesive patch is covered with an individual release member or patch. The release patch serves to protect the adhesive from being contaminated prior to use and from sticking to a surface other than the pant prior to use. However, grasping and removing four small pieces of release paper can be difficult and tedious.

Accordingly, it would be desirable to provide an incontinence pad with a release strip which is easy to grasp and remove.

### SUMMARY OF THE INVENTION

The present invention is directed to an incontinence pad which is to be secured to a pant. The incontinence pad has a pair of end edges and a pair of side edges. The incontinence pad comprises a liquid pervious topsheet; a liquid impervious backsheet secured to the topsheet; and an absorbent core intermediate the topsheet and the backsheet. At least a pair of securement patches are positioned adjacent one of the end edges of the incontinence pad. The patches are spaced apart from one another. A unitary release strip bridges the securement patches. Most preferably, a pair of securement patches are positioned adjacent each end edge of the incontinence pad and a unitary release strip bridges each pair of securement patches.

The securement patches may have a shape selected from the group consisting of rectangular, round, square, triangular, oval, oblong, diamond, pentagonal, and chevron.

The securement patches preferably comprise an adhesive patch. However, the securement patches may comprise a hook fastening material, or a combination adhesive patch and hook fastening material.

### BRIEF DESCRIPTION OF THE DRAWINGS

While the specification concludes with claims particularly pointing out and distinctly claiming the subject matter which is regarded as the present invention, it is believed that the description will be better understood from the following descriptions which are taken in conjunction with the accompanying drawings in which like designations are used to designate substantially identical elements.
Figure 1 is a plan view of an incontinence pad of the present invention having portions cut away to reveal the underlying structure with the garment-facing surface of the pad facing the viewer.
Figure 2 is a plan view of an alternative embodiment of an incontinence pad of the present invention with the garment-facing surface of the pad facing the viewer.
Figure 3 is a plan view of an alternative embodiment of an incontinence pad of the present invention with the garment-facing surface of the pad facing the viewer.

### DETAILED DESCRIPTION OF THE INVENTION

As used herein, the term "absorbent article" refers to devices which absorb and contain body exudates, and more specifically, refers to devices which are placed against or in proximity to the body of the wearer to absorb and contain the various exudates discharged from the body. The term "disposable" is used herein to describe absorbent articles which generally are not intended to be laundered or otherwise restored or reused as an absorbent article (i.e., they are intended to be discarded after a single use and, preferably, to be recycled, composted or otherwise disposed of in an environmentally compatible manner). As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

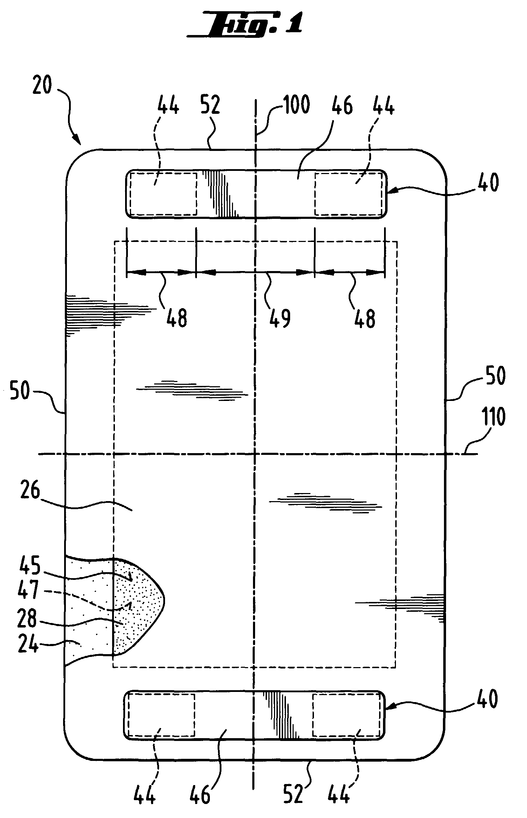

A preferred embodiment of an absorbent article of the present invention is the disposable incontinence pad (20), shown in Figure 1. As used herein, the term "incontinence pad" refers to an absorbent article worn by incontinent persons in combination with an elasticized pant. The incontinence pad (20) has a length dimension of at least about 300 mm and a width dimension of at least about 100 mm. In order to function properly as an incontinence device, the incontinence pad must be able to absorb at least about 300 grams of fluid, such as urine. The absorbent capacity is determined according to the standard ISO 11 948-1 method.

Figure 1 is a plan view of the incontinence pad (20) of the present invention in a flat-out, state with portions of the structure being cut-away to more clearly show the construction of the incontinence pad (20). The portion of the incontinence pad (20) which faces the wearer is oriented towards the viewer. As shown in Figure 1, the incontinence pad (20) preferably comprises a liquid pervious topsheet (24); a liquid impervious backsheet (26); an absorbent core (28), which is positioned between the topsheet (24) and the backsheet (26). The periphery of the incontinence pad (20) is defined by the outer edges of the pad (20) in which the side or longitudinal edges (50) run generally parallel to the longitudinal centerline (100) of the pad (20) and the end edges (52) run between the longitudinal edges (50) generally parallel to the lateral centerline (110) of the pad (20).

The backsheet (26) is generally that portion of the pad (20) positioned adjacent the garment facing surface (45) of the absorbent core (28) which prevents the exudates absorbed and contained therein from soiling other articles which contact the pad (20), such as the pant to which the pad is secured or other clothing. In preferred embodiments, the backsheet (26) is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by British Petroleum, Wasserburg Germany, sold under the trade name BPC "44118 LLD" COL 0022.

Other suitable backsheet materials may include breathable materials which permit vapors to escape from the pad (20) while still preventing exudates from passing through the backsheet (26). Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746, published on June 22, 1995 in the name of E. I. DuPont and copending U.S. Patent Application Serial No. 08/744,487, filed on November 6, 1996 in the name of Curro. Other breathable backsheets including nonwoven webs and apertured formed films are described in U.S. Pat. No. 5,571,096 issued to Dobrin et al. on November 5, 1996. Each of these references is hereby incorporated by reference herein.

The backsheet (26) may be joined to the topsheet (24), the absorbent core (28) or any other element of the pad (20) by any attachment means known in the art. For example, the attachment means may include a uniform continuous layer of adhesive, a patterned layer of adhesive, an array of separate lines, spirals, or spots of adhesive, or combinations thereof. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include several lines of adhesive filaments which are swirled into a spiral pattern, as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Adhesives which have been found to be satisfactory are manufactured by H. B. Fuller Company of St. Paul, Minnesota and marketed as HL-1258. Alternatively, the attachment means may comprise heat bonds, pressure bonds, ultrasonic bonds, dynamic mechanical bonds, or any other suitable attachment means or combinations of these attachment means as are known in the art.

The topsheet (24) is preferably positioned adjacent the body surface (47) of the absorbent core (28) and may be joined thereto and/or to the backsheet (26) by any attachment means known in the art. Suitable attachment means are described above with respect to means for joining the backsheet (26) to other elements of the pad (20). In one preferred embodiment of the present invention, the topsheet (24) and the backsheet (26) are joined directly to each other in some locations and are indirectly joined together in other locations by directly joining them to other elements of the pad (20).

The topsheet (24) is preferably compliant, soft feeling, and non-irritating to the wearer's skin. Further, at least a portion of the topsheet (24) is liquid pervious, permitting liquids to readily penetrate through its thickness. A suitable topsheet (24) may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the absorbent assemblies include fibers, the fibers may be spunbond, carded, wet-laid, meltblown, hydroentangled, or otherwise processed as is known in the art. One suitable topsheet (24) comprising a web of staple length polypropylene fibers is manufactured by Fiberweb Norrkoping Sweden, sold under trade name "E" (96), "C" (96), "F" (96).

Suitable formed film topsheets are described in U.S. Pat. No. 3,929,135, entitled "Absorptive Structures Having Tapered Capillaries", which issued to Thompson on December 30, 1975; U.S. Pat. No. 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel, et al. on August 3, 1982; U.S. Pat. No. 4,463,045 entitled "Macroscopically Expanded Three-Dimensional Plastic Web Exhibiting Non-Glossy Visible Surface and Cloth-Like Tactile Impression", which issued to Ahr, et al. on July 31, 1984; and U.S. Pat. No. 5,006,394 "Multilayer Polymeric Film" issued to Baird on April 9, 1991. Other suitable topsheets 30 are made in accordance with U.S. Pat. Nos. 4,609,518 and 4,629,643 which issued to Curro et al. on September 2, 1986 and December 16, 1986, respectively, and both of which are incorporated herein by reference. Such formed films are available from The Procter & Gamble Company of Cincinnati, Ohio as "DRI-WEAVE" and from Tredegar Corporation of Terre Haute, Indiana as "CLIFF-T."

Preferably, the topsheet (24) is made of a hydrophobic material or is treated to be hydrophobic in order to isolate the wearer's skin from liquids contained in the absorbent core (28). If the topsheet (24) is made of a hydrophobic material, preferably at least the upper surface of the topsheet (24) is treated to be hydrophilic so that liquids will transfer through the topsheet more rapidly. This diminishes the likelihood that body exudates will flow off the topsheet (24) rather than being drawn through the topsheet (24) and being absorbed by the absorbent core (28). The topsheet (24) can be rendered hydrophilic by treating it with a surfactant or by incorporating a surfactant into the topsheet. Suitable methods for treating the topsheet (24) with a surfactant include spraying the topsheet (24) material with the surfactant and immersing the material into the surfactant. A more detailed discussion of such a treatment and hydrophilicity is contained in U.S. Pat. No. 4,988,344 entitled "Absorbent Articles with Multiple Layer Absorbent Layers" issued to Reising, et al. on Jan. 29, 1991 and U.S. Pat. No. 4,988,345 entitled "Absorbent Articles with Rapid Acquiring Absorbent Cores" issued to Reising on Jan. 29, 1991. A more detailed discussion of some suitable methods for incorporating surfactant in the topsheet can be found in U.S. Statutory Invention Registration No. H1670, published on July 1, 1997 in the names of Aziz et al. Each of these references is hereby incorporated by reference herein.

Any portion of the topsheet (24) may be coated with a lotion as is known in the art. Examples of suitable lotions include those described in U.S. Pat. No. 5,607,760 entitled "Disposable Absorbent Article Having A Lotioned Topsheet Containing an Emollient and a Polyol Polyester Immobilizing Agent" which issued to Roe on March 4, 1997; U.S. Pat. No. 5,609,587 entitled "Diaper Having A Lotioned Topsheet Comprising A Liquid Polyol Polyester Emollient And An Immobilizing Agent" which issued to Roe on March 11, 1997; U.S. Pat. No. 5,635,191 entitled "Diaper Having A Lotioned Topsheet Containing A Polysilozane Emollient" which issued to Roe et al. on June 3, 1997; and U.S. Pat. No. 5,643,588 entitled "Diaper Having A Lotioned Topsheet" which issued to Roe et al. on July 1, 1997. The topsheet may also include or be treated with antibacterial agents, some examples of which are disclosed in PCT Publication No. WO 95/24173 entitled "Absorbent Articles Containing Antibacterial Agents in the Topsheet For Odor Control" which was published on September 14, 1995 in the name of Johnson. Further, the topsheet (24), the backsheet (26) or any portion of the topsheet or backsheet may be embossed and/or matte finished to provide a more cloth like appearance.

The absorbent core (28) may comprise any absorbent material which is generally compressible, conformable, non-irritating to the wearer's skin, and capable of absorbing and retaining liquids such as urine and other certain body exudates. The absorbent core (28) may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The configuration and construction of the absorbent core (28) may also be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures). However, the total absorbent capacity of the absorbent core (28) should be compatible with the design loading and the intended use of the pad (20).

Exemplary absorbent structures for use as the absorbent assemblies are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735, entitled "High Density Absorbent Members Having Lower Density and Lower Basis Weight Acquisition Zones", issued to Alemany et al. on May 30, 1989; U.S. Patent 4,888,231 entitled "Absorbent Core Having A Dusting Layer" issued to Angstadt on December 19, 1989; U.S. Pat. No. 5,137,537 entitled "Absorbent Structure Containing Individualized, Polycarboxylic Acid Crosslinked Wood Pulp Cellulose Fibers" which issued to Herron et al. on August 11, 1992; and U.S. Patent 5,147,345 entitled "High Efficiency Absorbent Articles For Incontinence Management" issued to Young et al. on September 15, 1992; U.S. Pat. No. 5,342,338 entitled "Disposable Absorbent Article For Low-Viscosity Fecal Material" issued to Roe on August 30, 1994. Each of these patents is incorporated herein by reference.

The pad 20 also includes a securement system (40) for securing the pad (20) in a pant. The securement system (40) preferably comprises at least a pair of patches (44) positioned on the backsheet (26) adjacent one end edge (52) of the pad (20). Even more preferably, the securement system (40) comprises a pair of patches (44) positioned on the backsheet (26) adjacent each end edge (52) of the pad (20). As can be seen in Figure 1 the patches (44) are spaced apart from one another. It has been found that spacing the patches (44) from one another provides the needed securement of the pad (20) to a pant while maintaining the desired freedom of movement for the pad/pant system to ensure wearer comfort. For example, it has been found that securing the entire end edge (52) of the pad (20) to a pant provides very good securement, but unnecessarily restricts the movement of the pant and also adds unnecessarily to the cost of the product.

The patches (44) preferably comprise a pressure sensitive adhesive. A suitable pressure sensitive adhesive is sold by 3M Health Care, St. Paul, MN 55144-1000.

The patches (44) may be rectangular in shape having a size of about 50 millimeters x 30 millimeters. However, the patches (44) may have other shapes such as but not limited to round, square, triangular, star, oval, oblong, diamond, pentagonal, and chevron. The patches (44) may also be colored for ease of identification.

To prevent contamination of the adhesive patches (44) and to prevent the patches from sticking to the pant prematurely the adhesive patches (44) are covered with a unitary release strip (46). The release strip (46) may be made of poly film (e.g., silicone coated polyethylene film), kraft paper, calendered paper, or any other materials well known in the art for such purposes.

Preferably the face of the release strip (46) which contacts the adhesive patches (44) has a release coating, such as silicone, to facilitate the removal of the release strip (46) from the patches (44). Suitable release coatings are marketed by Akrosil of Menasha, Wisconsin as Silox 4R/O and as Silox C1S. If desired, the outwardly oriented face of the release strip (46) may further comprise indicia, such as advertising or usage instructions thereon. In addition or alternatively, the release strip 46 may be colored for easy identification. In addition, the release strip 46 in the front of the pad (20) may have one color while the release strip (46) in the back of the pad (20) may have another color.

The unitary release strip (46) bridges a pair of patches (44). A component, such as a release strip (46) is considered unitary if it cannot be divided or disassembled without tearing or unintended gross separation. It is not necessary that a unitary component be made of a single material but, rather that such component cannot be disassembled from and subsequently reassembled into the original configuration. Components are considered to be "bridged" if they are connectively spanned by an independent component.

It is important that the unitary release strip (46) be conveniently and easily manipulated by the wearer. With a pad (20) having the unitary release strip (46) according to this invention, the pad (20) can be positioned on the wearer, the pant can then be pulled over the pad, and then the caregiver or wearer can reach inside the pant grab the central portion (47) of the release strip (46) which is unattached to any portion of the pad (20), and peel off the unitary release strip (46) removing the end portions (48) of the release strip (46) from the patches (44) thereby uncovering both patches (44) with a single motion. The end portions (48) are generally those portions of the release strip (46) which are secured to the patches (44) prior to removal of the release strip (46).

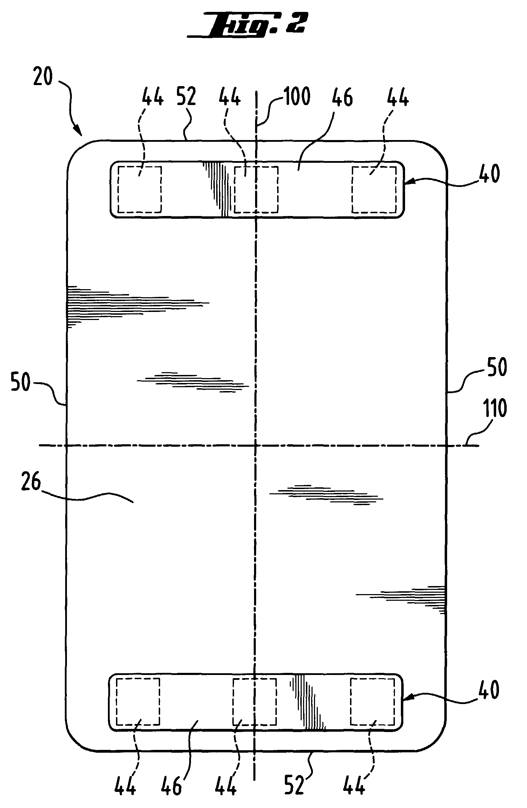

In an another embodiment shown in Figure 2, the securement system (40) comprises three patches (44) positioned on the backsheet (26) adjacent each end edge (52) of the pad (20). As can be seen in Figure 2 the patches (44) are spaced apart from one another. The unitary release strip (46) bridges all three patches (44). The addition of the third patch (44) between the two outer patches helps to maintain the release strip (46) in place while the pant is pulled on over the pad (20) during application to the wearer.

In the embodiment shown in Figure 1, the patches (44) have a width dimension equal to that of the release strip (46). In the embodiment shown in Figure 2, the patches (44) have a width dimension less than that of the release strip (46).

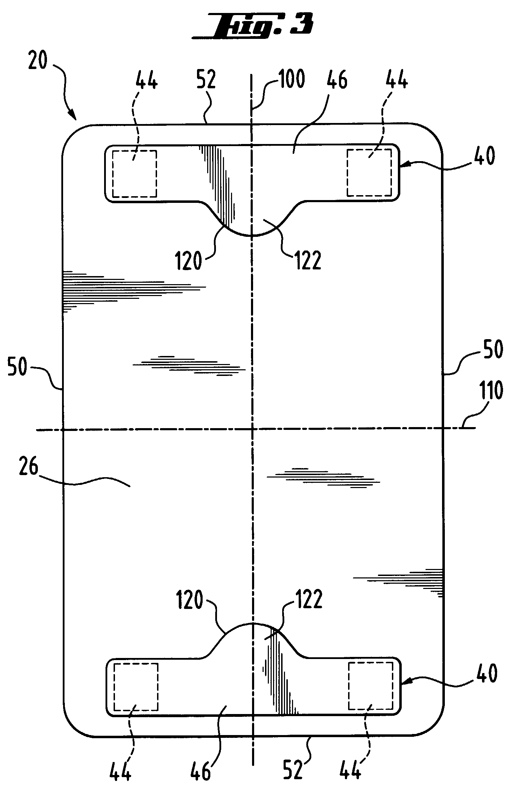

In another embodiment shown in Figure 3, the securement system (40) comprises a pair of patches (44) positioned on the backsheet (26) adjacent each end edge (52) of the pad (20). The unitary release strip (46) comprises a means (120) for initiating removal of the release strip (46) at or near its center, preferably at the longitudinal centerline (100). This may be accomplished, for example, by any appendage to the release strip (46) which enables it to be grasped near the longitudinal centerline (100) of the pad (20). For example, the release strip (46) may be shaped to have a tab (122) which the user may grasp.

The patches (44) may comprise a hook fastening material or combination hook fastening material and adhesive although any other known fastening means is generally acceptable. Suitable hook fastening materials are disclosed in U.S. Patent 4,846,815 issued to Scripps on July 11, 1989; U.S. Patent 4,894,060 issued to Nestegard on January 16, 1990; U.S. Pat. No. 5,151,092 issued to Buell on September 9, 1992; and U.S. Pat. No. 5, 221,274 issued to Buell on June 22, 1993. The fastening system may also provide a means for holding the article in a disposal configuration as disclosed in U.S. Pat. No. 4,963,140 issued to Robertson et al. on October 16, 1990. Each of these patents is incorporated herein by reference.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An incontinence pad (20) to be secured to a pant, said incontinence pad (20) having a pair of end edges (52) and a pair of side edges (50), said incontinence pad comprising
a liquid pervious topsheet (24);
a liquid impervious backsheet (26) secured to said topsheet;
an absorbent core (28) intermediate said topsheet and said backsheet;
at least a pair of securement patches (44) positioned adjacent one of said end edges of said incontinence pad, said securement patches being spaced from one another; and
characterized in that
a unitary release strip (46) bridges said securement patches.

2. The incontinence pad of claim 1 comprising a pair of securement patches positioned adjacent each end edge of said incontinence pad and a unitary release strip bridging each pair of securement patches.

3. The incontinence pad of claim 1 or claim 2 wherein said securement patches have a shape selected from the group consisting of rectangular, round, square, triangular, star, oval, oblong, diamond, pentagonal, and chevron.

4. The incontinence pad of any one of the preceding claims wherein said securement patches comprise an adhesive patch.

5. The incontinence pad of any one of claims 1, 2 or 3 wherein said securement patches comprise a hook patch.

6. The incontinence pad of any one of claims 1, 2 or 3 wherein said securement patches comprise a combination adhesive and hook patch.

7. The incontinence pad of any one of claims 1, 2 or 3 wherein said securement patches have a width dimension equal to a width dimension of said release strip.

8. The incontinence pad of any one of claims 1, 2 or 3 wherein said securement patches have a width dimension less than a width dimension of said release strip.

9. An incontinence pad (20) to be secured to a pant, said incontinence pad having a pair of end edges (52) and a pair of side edges (50), said incontinence pad comprising
a liquid pervious topsheet (24);
a liquid impervious backsheet (26) secured to said topsheet;
an absorbent core (28) intermediate said topsheet and said backsheet;
a pair of securement patches (44) positioned adjacent each end edge of said incontinence pad, said securement patches being spaced from one another; and
characterized in that
a unitary release strip (46) bridges said securement patches.

10. The incontinence pad of claim 9 wherein said securement patches have a shape selected from the group consisting of rectangular, round, square, triangular, oval, oblong, diamond, pentagonal, and chevron.

11. The incontinence pad of either claim 9 or claim 10 wherein said securement patches comprise an adhesive patch.

12. The incontinence pad of any one of claims 9, 10 or 11 wherein said securement patches comprise a hook patch.

13. The incontinence pad of any one of claims 9, 10 or 11 wherein said securement patches comprise a combination adhesive and hook patch.
